# EUROPEAN PATENT APPLICATION

(11) **EP 4 667 052 A2**
(43) Date of publication of application: **24.12.2025**
(21) Application number: 25213570.2
(22) Date of filing: 02.11.2022
(51) Int. Cl.: A61P 31/04

(54) **MEDICINAL COMPOSITION COMPRISING RIBOSE AND AMINO ACIDS**

(30) Priority: 20.12.2021 DE 102021133895
(62) Divisional of application: 22813909.3
(71) Applicant: Mee-hu Pharma GmbH, 4020 Linz (AT)
(72) Inventor: SCHMIED, Arnold, 4020 Linz (AT)
(74) Representative: Lorenz Seidler Gossel Part. mbB

(57) **Abstract**

The invention relates to a composition for use in a method of treating a bacterial infection selected from a bacterial urinary tract infection, a bacterial respiratory tract infection, a bacterial soft tissue infection and a bacterial bone infection in a patient, the composition comprising ribose and the amino acids glycine, alanine and glutamine in aqueous solution, the method comprising orally administering the composition to the patient additionally treated with an antibiotic.

## Description

The invention relates to a composition for use in a method of treating a bacterial infection selected from a bacterial urinary tract infection, a bacterial respiratory tract infection, a bacterial soft tissue infection and a bacterial bone infection in a patient.

Urinary tract infections are among the most common bacterial diseases worldwide. Community-associated prevalence is 0.7%. Healthcare-associated UTIs frequency among healthcare-associate infections is 12.9, 19.6 and 24% in the United States, Europe and developing countries, respectively. In urology departments, the prevalence is 5.1%. Urinary tract infections are often caused by both Gram-negative and Gram-positive bacteria, frequently by Escherichia Coli and Klebsiella pneumonia. High recurrence rate and increasing antimicrobial resistance among uropathogens threaten to greatly increase the burden of these infections.

The invention proposes a new treatment for a bacterial infection selected from a bacterial urinary tract infection, a bacterial respiratory tract infection, a bacterial soft tissue infection and a bacterial bone infection.

Specifically, the invention proposes a composition for use in a method of treating a bacterial infection selected from a bacterial urinary tract infection, a bacterial respiratory tract infection, a bacterial soft tissue infection and a bacterial bone infection in a patient, the composition comprising ribose and the amino acids glycine, alanine and glutamine in aqueous solution, the method comprising orally administering the composition to the patient that is co-treated with an antibiotic. Preferably, the infection treated is a bacterial urinary tract infection.

The ribose is preferably D-ribose. The alanine is preferably racemic (D/L)-alanine. The glutamine is preferably L-glutamine.

The concentration of ribose in the aqueous solution of the composition is preferably between 30 and 40 g/l. The concentration of glycine in the aqueous solution of the composition is preferably between 1.5 and 2.5 g/l. The concentration of alanine in the aqueous solution of the composition is preferably between 15 and 25 g/l. The concentration of glutamine in the aqueous solution of the composition is preferably between 20 and 30 g/l. The relative concentrations of the amino acids in the aqueous solution of the composition are preferably 1 : 8-12 : 10-15 (glycine : alanine : glutamine). The relative concentrations of the ribose and the amino acids glycine, alanine and glutamine in the aqueous solution of the composition are preferably 1 : 0.8-2.0 (ribose : amino acids). The volume of the aqueous solution of the composition is preferably 5 to 50 ml, preferably 10 to 30 ml.

In a preferred embodiment, the composition is administered to the patient twice or three times daily. A two times daily administration can be most preferred. In embodiments, the composition may be administered to the patient daily over a period of one to three weeks, preferably one to two weeks.

The amount of ribose administered to the patient per day is preferably between 1200 and 2400 mg. The amount of glycine administered to the patient per day is preferably between 60 and 150 mg. The amount of alanine administered to the patient per day is preferably between 600 and 1500 mg. The amount of glutamine administered to the patient per day is preferably between 800 and 1800 mg. The relative amounts of the amino acids administered to the patient are preferably 1 : 8-12 : 10-15 (glycine : alanine : glutamine). The relative amounts of the ribose and the amino acids glycine, alanine and glutamine administered to the patient are preferably 1 : 0.8-2.0 (ribose : amino acids).

The patient is preferably a human patient. In particular, the human patient can be an adult of more than 18 years.

In one embodiment, the composition can be used in treating infections caused by a bacterial strain having multiple resistance to antibiotics, in particular Escherichia Coli or Klebsiella Pneumonia bacterial strains having multiple resistance to antibiotics. It has previously been proposed that a composition comprising ribose, glycine, alanine and glutamine could have a beneficial effect in the treatment of infections going back to multiple antibiotic resistant bacteria in DE 10 2011 105 594 B4.

The method generally comprises treating the patient with an antibiotic in addition to co-treating the patient with the inventive composition. The inventive composition is intended to enhance the effect of the antibiotic. In one embodiment, the inventive composition is co-administered with every or every other administration of antibiotic. In another embodiment, the inventive composition is administered at least half an hour and at the most five hours prior every or every other administration of antibiotic. The rationale is that the composition may increase sensitivity of the bacteria to antibiotic treatment and that the composition should be allowed to act on the bacteria before antibiotic administration.

The antibiotic administered to the patient may be a penicillin, in particular ampicillin, a cephalosporin antibiotic, like Cefazolin, a fluoroquinolone-type antibiotic, like Ciprofloxacin, and a sulphonamide, like Sulfamethoxazole, possibly in combination with Trimethoprim.

The aqueous solution forming for the composition may be obtained by dissolving a mixed lyophilisate of the amino acids in an aqueous solution of ribose. The mixed lyophilisate can be obtained by lyophilizing a solution comprising the amino acids glycine, alanine and glutamine in an appropriate ratio.

Further details and advantages of the invention are described with reference to the following examples and figures. The figures show :
- Figure 1a:: a result plot for strain #1 and antibiotic #1 of the in vitro study described below;
- Figure 1b:: a result plot for strain #1 and antibiotic #2 of the in vitro study described below;
- Figure 1c:: a result plot for strain #1 and antibiotic #3 of the in vitro study described below;
- Figure 1d:: a result plot for strain #1 and antibiotic #4 of the in vitro study described below;
- Figure 2a:: a result plot for strain #2 and antibiotic #1 of the in vitro study described below;
- Figure 2b:: a result plot for strain #2 and antibiotic #2 of the in vitro study described below;
- Figure 2c:: a result plot for strain #2 and antibiotic #3 of the in vitro study described below;
- Figure 2d:: a result plot for strain #2 and antibiotic #4 of the in vitro study described below;
- Figure 3a:: a result plot for strain #3 and antibiotic #1 of the in vitro study described below;
- Figure 3b:: a result plot for strain #3 and antibiotic #2 of the in vitro study described below;
- Figure 3c:: a result plot for strain #3 and antibiotic #3 of the in vitro study described below;
- Figure 3d:: a result plot for strain #3 and antibiotic #4 of the in vitro study described below;
- Figure 4a:: a result plot for strain #4 and antibiotic #1 of the in vitro study described below;
- Figure 4b:: a result plot for strain #4 and antibiotic #2 of the in vitro study described below;
- Figure 4c:: a result plot for strain #4 and antibiotic #3 of the in vitro study described below;
- Figure 4d:: a result plot for strain #4 and antibiotic #4 of the in vitro study described below; and

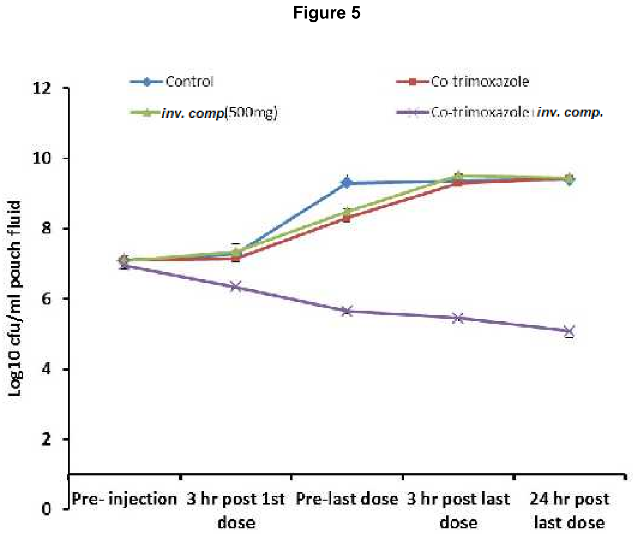

- Figure 5:: a result plot for in vivo study #2.

### Composition:

An aqueous composition was obtained by combining and mixing one vial of lyophilisate by the description of Table 1 below with two ampoules of a 3.5% D-ribose solution by the description of Table 2 below.

**Table 1: Lyophilisate**

| Description | White to off white powder in 30 ml glass bottle Type 1 with rubber closure and HDPE white cap |
|---|---|
| Average filling weight | 940 mg ± 10% |
| Water content | not more than 5.0 % |
| Identification test by HPLC: | |
| DL-alanine | Positive |
| L-glutamine | Positive |
| Glycine | Positive |
| Assay by HPLC for: | |
| DL-alanine | 90-110% of the labelled amount (360-440 mg) |
| L-glutamine | 90-110% of the labelled amount (450-550 mg) |
| Glycine | 90-110% of the labelled amount (36-44 mg) |

| Microbiological Analysis | |
|---|---|
| Total bacterial count | NMT 1000 CFU/g |
| Total fungal count | NMT 100 CFU/g |

| Pathogenic microorganism | |
|---|---|
| - Escherichia Coli | Free |
| - Salmonella | Free |
| - Staphylococcus aureus | Free |

**Table 2: 3.5% D-ribose solution**

| Description | A clear, colourless and odourless solution packed in polypropylene plastic ampoule |
|---|---|
| Total filling volume | NLT 10 ml |
| HPLC identification of D-ribose | Positive (conform) |
| HPLC assay of D-ribose | 90-110% of the labelled amount |

| Microbiological Analysis | |
|---|---|
| Total bacterial count | NMT 1000 CFU/g |
| Total fungal count | NMT 100 CFU/g |

| Pathogenic microorganism | |
|---|---|
| - Escherichia Coli | Free |
| - Salmonella | Free |
| - Staphylococcus aureus | Free |

After combining and mixing one vial of lyophilisate and two vials of ribose solution, a liquid composition by the description of Table 3 was obtained.

**Table 3: Mixed solution**

| Description | Transparent to faint yellow solution |
|---|---|
| Average volume content | 20 ml ±10% |
| Clarity | Clear |
| Identification test by HPLC: | |
| DL-alanine | Positive |
| L-glutamine | Positive |
| D-ribose | Positive |
| Glycine | Positive |
| Assay by HPLC for: | |
| DL-alanine | 90-110% of stated amount |
| L-glutamine | 90-110% of stated amount |
| D-ribose | 90-110% of stated amount |
| Glycine | 90-110% of stated amount |

The obtained composition was subjected to a number of in vitro and in vivo mouse model tests.

### In vitro study:

The effect of the composition on antibiotic sensitivity was tested in vitro in four resistant bacterial strains.

The study was performed in agreement with the description of Ostrosky E. A., Mizumoto M.K., Lima M. E. L., Kaneko T. M., Nishikawa S. O., Freitas B. R. Rev Bras Farmacogn (2008) 18:301-307.

Each purified bacterial strain was inoculated into culture of nutrient broth and incubated overnight (18-20 hr), then bacterial suspension was prepared and turbidities adjusted to 0.2-0.25 at 360 nm. The concentrations of antibiotics used with the bacterial strains during the eight days of treatments were shown in Table 1. The panel of different treatments of the inventive composition and antibiotics against resistant strains in presence of different is detailed in Table 2.

**Table 1**

| | Ampicillin "AB #1" [µg/ml] | Cefalozin "AB #2" [mg/ml] | Trimethoprim/ Sulphamethoxazole "AB #3" [mg/ml] | Ciproxloxazin "AB #4" [µg/ml] |
|---|---|---|---|---|
| MRSA L2-16 ST 347 "Strain #1" | 0.4 | 0.97 | S 0.288 / T 0.057 | 2.0 |
| MRSA L2-15 ST 13DSMZ46320 "Strain #2" | 5.0 | 0.97 | S 2.48 / T 0.496 | 2.0 |
| ESBL2-3 Kleb. Pneu. "Strain #3" | 3.0 | 1.6 | S 1.24 / T 0.25 | 2.0 |
| ESBL2-1 E. Coli "Strain #4" | 8.0 | 6.2 | S 2.48 / T 0.496 | 31.2 |

**Table 2**

| Strain | Antibiotic | Treatment Panel | Group # |
|---|---|---|---|
| each of Table 1 | each of Table 1 | Muller Hinton Broth (1.0ml) | #1 |
| | | Muller Hinton Broth (0.5ml) + Distilled water (0.5ml containing antibiotic) | #2 |
| | | Muller Hinton Broth (0.5ml) + inventive composition (0.5ml containing antibiotic) | #3 |
| | | Muller Hinton Broth (0.5ml) + inventive composition (0.5ml) | #4 |

All vials were incubated at 37°C. After an onset of 24 hours, the bacterial population in each vial was monitored daily using a spectrophotometer at 630nm.

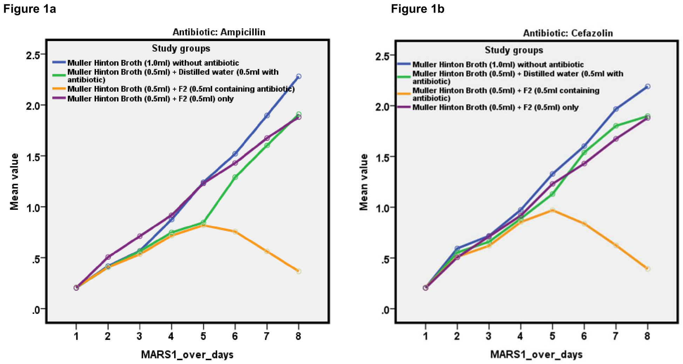

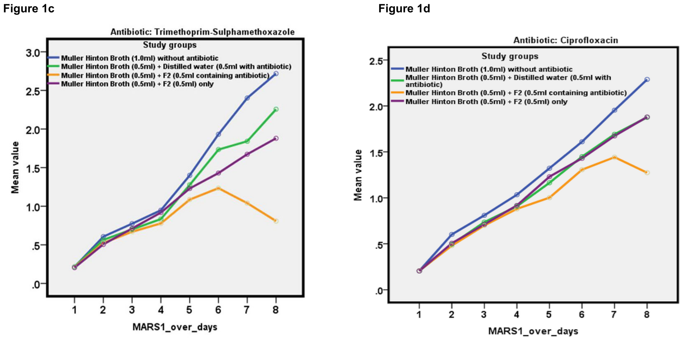

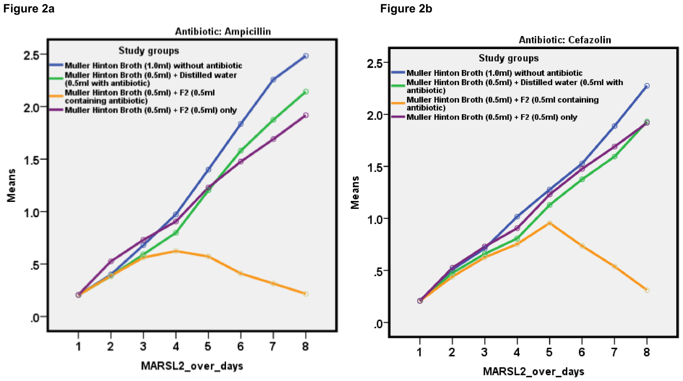

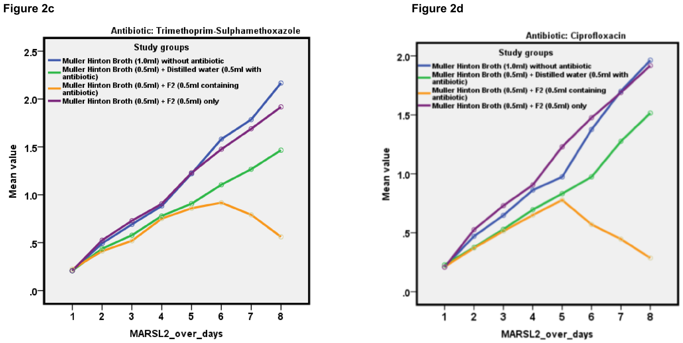

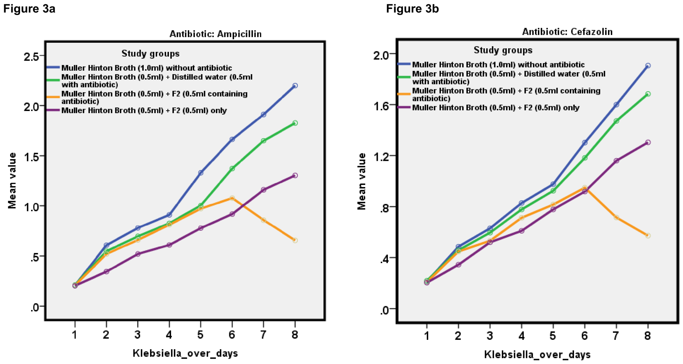

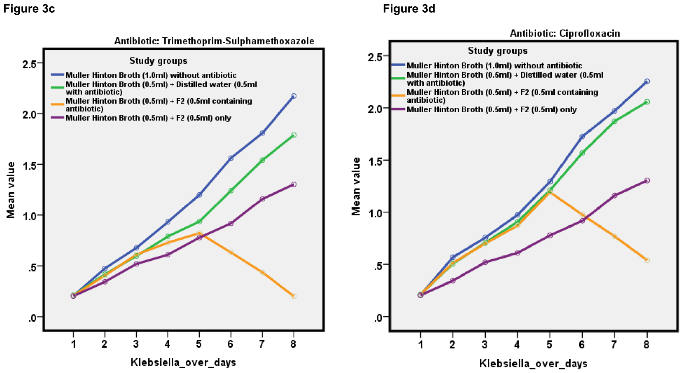

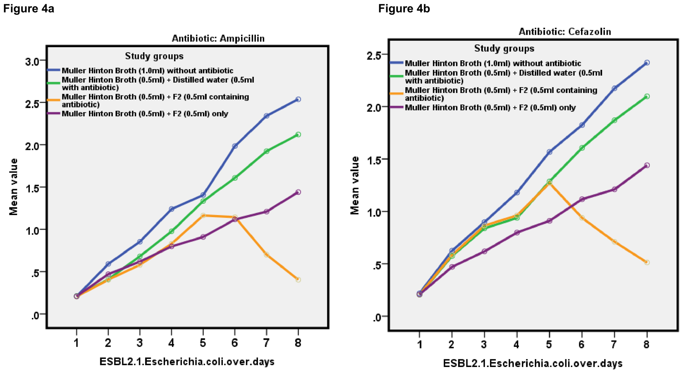

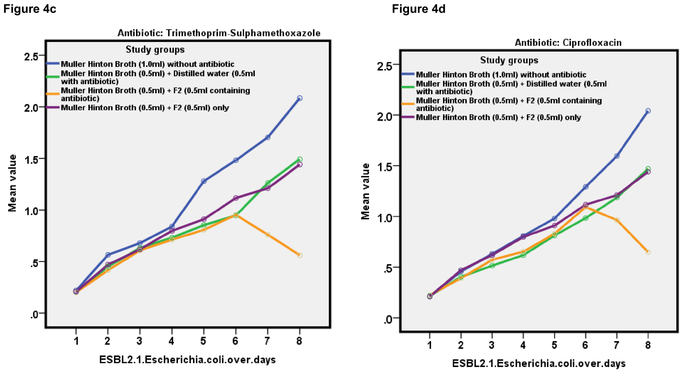

The results are shown in Figures 1a to 4d. Specifically, Figure 1a shows the results for strain #1 and antibiotic #1. Figure 1b shows the results for strain #1 and antibiotic #2. Figure 1c shows the results for strain #1 and antibiotic #3. Figure 1d shows the results for strain #1 and antibiotic #4. Figure 2a shows the results for strain #2 and antibiotic #1. Figure 2b shows the results for strain #2 and antibiotic #2. Figure 2c shows the results for strain #2 and antibiotic #3. Figure 2d shows the results for strain #2 and antibiotic #4. Figure 3a shows the results for strain #3 and antibiotic #1. Figure 3b shows the results for strain #3 and antibiotic #2. Figure 3c shows the results for strain #3 and antibiotic #3. Figure 3d shows the results for strain #3 and antibiotic #4. Figure 4a shows the results for strain #4 and antibiotic #1. Figure 4b shows the results for strain #4 and antibiotic #2. Figure 4c shows the results for strain #4 and antibiotic #3. Figure 4d shows the results for strain #4 and antibiotic #4.

It is apparent from the graphs that the co-treated groups show a significant reduction of bacterial growth a few days into treatment, whereas the only antibiotic treatment shows no significant effect over the control. Also the only treatment with the inventive composition shows no significant effect over control. This suggests that the inventive composition aids in making the resistant bacterial more sensitive to antibiotic attack.

### N vivo animal study #1:

A group of Sprague Dawley rats was infected with antibiotic resistant staphylococcus aureus (MRSA L2-15 ST 13DSMZ46320) strains (intraperitoneal, 1x10⁶ CFU/Rat enhanced with 6% hog mucin type III) and subgroups of 10 rats (5 males and 5 females) were left either untreated, treated with 10 mg/kg/day (p.o.) of the antibiotic Rifampicin for 10 successive days, co-treated with 10 mg/kg/day (p.o.) of the antibiotic Rifampicin and 250 mg/kg/day (p.o.) of the inventive composition (the 250 mg referring to the weight of the lyophilisate of Table 1 contained in the amount of mixed solution administered) for 10 successive days, or co-treated with 10 mg/kg/day (p.o.) of the antibiotic Rifampicin and 500 mg/kg/day (p.o.) of the inventive composition and for 10 successive days. Survival after 10 days was recorded.

In the untreated group, no animals survived after 10 days. In the group treated with the antibiotic alone, two of the ten animals survived. In the co-treated group receiving 250 mg/kg/day of the inventive composition, seven of the ten animals survived. In the co-treated group receiving 500 mg/kg/day of the inventive composition, all animals survived.

### In vivo animal study #2:

Male Wistar rats, weighing 200-250 g, were infected with a resistant strain of E. Coli (O157.H7) using the granuloma pouch model. Specifically, pouches were produced by injecting 30 ml of sterile filtered air followed by injection of 0.5 ml of 0.5% croton oil in sesame seed oil deep into the loose connective dorsal tissue. Two days later, approximately 10 ml of air were withdrawn, and after 2 more days, 2 ml of 1% peptone and 0.35% agar in saline solution were injected in the pouch. On day 10, rats were infected with 106 CFU of E. coli suspended in 1.5 ml of THB containing 2.5% gastric mucin and 0.35% agar.

The rats were divided in groups of five rats, and the following drugs, either alone or concurrently administered, were administered every 12 h for three consecutive days, making a total of six doses, starting 24 h after infection: co-trimoxazole (Septrin, GlaxoSmithKline) 30 mg/kg (i.p) and the inventive composition 500 mg/kg (p.o) (the 500 mg referring to the weight of the lyophilisate of Table 1 contained in the amount of mixed solution administered). A control group of five untreated control animals received six oral doses of placebo and intraperitoneal injections of saline at the same intervals.

Samples (0.5 mL) of pouch fluid were withdrawn from each animal just before treatment (0 time), 3 h after the first dose, just before the last dose, 3 and 24 h after the last dose. Six tenfold dilutions of the pouch fluid were made in 1% peptone in saline and 10µl of each dilution and of undiluted pouch fluid were spread in duplicate over the surface of blood agar plates, which were incubated at 37°C for 24h. The results represent the average values obtained from all the animals in the same group at the same intervals and expressed as log 10 CFU/ml. One-way ANOVA with Bonferroni's Multiple Comparison Test was used to identify differences significant at the 0.05 level, following a standard analysis of variance.

The treatment efficacy in terms of bacterial counts is shown in the Figure. At all time intervals there were no significant differences between the control and the inventive composition alone and the cotrimoxazole alone treated groups. A 0.93 Log decline (p≤ 0.01) in bacterial counts for the group tested with the inventive composition plus cotrimoxazole was observed as early as after 3 hours from the first dose. This declining effect increased with consecutive doses to reach 3.6 Log declining (p≤ 0.01) before the last (sixth) dose and persisted 24 h after the last dose with 4.3 Log declining in comparison to the control group (p≤ 0.01). Even when the difference from the initial count was used as the response measure, the group treated with cotrimoxazole and the inventive composition was significantly below the control group at all the time points.

### Clinical studies:

A phase II clinical study design that is intended to be carried out will encompass a number of patients with bacterial urinary tract infection. The patients will be treated with a combined administration of the inventive composition (2 times daily administration of 20 ml of the combined solution according to Table 3 above) and appropriate antibiotic administration over the course of 10 days. Results will be evaluated and benchmarked against treatment with antibiotic alone.

Specifically, the study design is as follows.

### Phase: II

### Indication: Recurrent Urinary Tract Infection

### Objectives:

Primary objective: Evaluation of the inventive composition's safety and evaluation of its effect, in combination with antimicrobial treatment, on urine culture (microbiological cure, no microbial growth on 24-48 hrs culture).

Secondary objective: Evaluation of the inventive composition's effect, in combination with antimicrobial treatment, on disease related symptoms (clinical cure, disappearance of symptoms and signs).

Design: randomized, double blind, placebo controlled multi-centre study. Recurrent urinary tract infection patients will blindly receive the inventive composition, or matching placebo, orally, twice daily, for 10 days, adjunct to the empirical antibiotic.

Population: Male and female patients over 18 years old diagnosed with recurrent urinary tract infection. Sample size: 100 patients from each centre.

Dosage schedule: All patients will be given the following:
1. Trimethoprim-sulfamethoxazole 160/800 mg, twice daily, every 12 hours, orally, for 10 days.
2. The inventive composition, or placebo (according to randomization), twice daily, every 12 hours, orally, for 10 days.

Efficacy Data:
- Complete response status: is clinical cure (disappearance of symptoms and signs) and microbiological cure (negative urine culture), 5-7 days after completion of treatment.
- Incomplete response status: is clinical cure (disappearance of symptoms and signs) with positive bacterial culture, 5-7 days after completion of treatment.
- Failure status: is no clinical response, 5- days after starting of treatment.
- Recurrence: is development of new symptoms of urinary tract infection in patients with previously clinical and microbiological cure plus positive urine cultures; 60 ±10 days from the first day of the study drug administration.

### Safety Data:

Adverse events reported by the subject or observed by the investigator.

### Statistical:

The primary efficacy analysis will use confidence procedures:
The two-sided confidence interval will be calculated for responder rate (e.g. Clopper and Pearson method). Paired t-test will be applied as a secondary efficacy analysis. Adverse events will be summarized in frequency tables, which will be sorted by body systems. Frequency tables by treatment group will be provided for all treatment emergent adverse event (TEAEs), and all possibly related TEAEs, and for all on-treatment adverse events. They will be broken down by the intensity of adverse events pre-treatment and post-treatment adverse events will be provided in subject listings. P value will be used to identify possibly important adverse events. Fisher's exact test will be considered for the treatment group comparisons of adverse events frequencies.

### Study Duration:

The duration of this study is expected to be 18 months. The duration of therapy is 10 days followed by 8 weeks follow up for each patient.

## Claims

1. A composition for use in a method of treating a bacterial infection selected from a bacterial urinary tract infection, a bacterial respiratory tract infection, a bacterial soft tissue infection and a bacterial bone infection in a patient, the composition comprising ribose and the amino acids glycine, alanine and glutamine in aqueous solution, the method comprising orally administering the composition to the patient that is co-treated with an antibiotic.

2. The composition for use according to claim 1, wherein the ribose is D-ribose and/or wherein the alanine is racemic (D/L)-alanine and/or wherein the glutamine is L-glutamine.

3. The composition for use according to any preceding claim, wherein
the concentration of ribose in the aqueous solution of the composition is between 30 and 40 g/l; and/or
the concentration of glycine in the aqueous solution of the composition is between 1.5 and 2.5 g/l; and/or
the concentration of alanine in the aqueous solution of the composition is between 15 and 25 g/l; and/or
the concentration of glutamine in the aqueous solution of the composition is between 20 and 30 g/I.

4. The composition for use according to any preceding claim, wherein the relative concentrations of the amino acids in the aqueous solution of the composition are 1 : 8-12 : 10-15 (glycine : alanine : glutamine).

5. The composition for use according to any preceding claim, wherein the relative concentrations of the ribose and the amino acids glycine, alanine and glutamine in the aqueous solution of the composition are 1 : 0.8-2.0 (ribose : amino acids).

6. The composition for use according to any preceding claim, wherein the volume of the aqueous solution of the composition is 10 to 30 ml.

7. The composition for use according to any preceding claim, wherein the composition is administered to the patient twice or three times daily and/or wherein the composition is administered to the patient daily over a period of one to three weeks.

8. The composition for use according to any preceding claim, wherein
the amount of ribose administered to the patient per day is between 1200 and 2400 mg; or
the amount of glycine administered to the patient per day is between 60 and 150 mg; or
the amount of alanine administered to the patient per day is between 600 and 1500 mg; or
the amount of glutamine administered to the patient per day is between 800 and 1800 mg.

9. The composition for use according to any preceding claim, wherein the relative amounts of the amino acids administered to the patient are 1 : 8-12 : 10-15 (glycine : alanine : glutamine).

10. The composition for use according to any preceding claim, wherein the relative amounts of the ribose and the amino acids glycine, alanine and glutamine administered to the patient are 1 : 0.8-2.0 (ribose : amino acids).

11. The composition for use according to any preceding claim, wherein antibiotic co-administered to the patient may be a penicillin, in particular ampicillin, a cephalosporin antibiotic, like Cefazolin, a fluoroquinolone-type antibiotic, like Ciprofloxacin, and a sulphonamide, like Sulfamethoxazole, possibly in combination with Trimethoprim.

12. The composition for use according to any preceding claim, wherein the infection is a urinary tract infection.

13. The composition for use according to any preceding claim, wherein the infection is caused by bacterial strains having multiple resistance to antibiotics, in particular Escherichia Coli or Klebsiella Pneumonia having multiple resistance to antibiotics.

14. The composition for use according to any preceding claim, wherein the composition is co-administered with every or every other administration of antibiotic.

15. The composition for use according to any one of claims 1-13, wherein the composition is administered at least half an hour and at the most five hours prior every or every other administration of antibiotic.
